Europäisches Patentamt

European Patent Office

Office européen des brevets

(19)

(11) Publication number: **0 171 749**
**B1**

# EUROPEAN PATENT SPECIFICATION

(12)

(45) Date of publication of patent specification: **04.01.89**

(51) Int. Cl.⁴: **A 61 M 1/00, A 61 M 1/34**

(21) Application number: **85109935.8**

(22) Date of filing: **07.08.85**

(54) **Apparatus for separating blood plasma, and apparatus therefor.**

(30) Priority: **07.08.84 JP 164218/84**

(43) Date of publication of application:
**19.02.86 Bulletin 86/08**

(45) Publication of the grant of the patent:
**04.01.89 Bulletin 89/01**

(84) Designated Contracting States:
**BE DE FR GB SE**

(56) References cited:
**EP-A-0 048 901**
**EP-A-0 085 016**
**EP-A-0 112 152**
**EP-A-0 131 529**
**WO-A-81/02979**
**GB-A-2 112 293**
**US-A-3 064 647**

(73) Proprietor: **TERUMO KABUSHIKI KAISHA**
**trading as TERUMO CORPORATION**
**44-1, 2-chome, Hatagaya Shibuya-Ku**
**Tokyo 151 (JP)**

(72) Inventor: **Watanabe, Masaharu**
**Sharumu Dai-Ni Seisekisakuragaoka 204**
**2-1, Sekido Tama-shi Tokyo (JP)**
Inventor: **Osawa, Takaaki**
**51, Kuroda**
**Fujinomiya-shi Shizuoka-ken (JP)**

(74) Representative: **Henkel, Feiler, Hänzel & Partner**
**Möhlstrasse 37**
**D-8000 München 80 (DE)**

EP 0 171 749 B1

**Description**

Background of the invention
1. Field of the invention:
This invention relates to an apparatus wherein a membrane-type blood plasma separator is used to separate blood plasma without continuously circulating blood externally of a donor, yet at an efficiency approximately equivalent to that of a continuous external circulation system by adopting a semicontinuous system in which blood is collected and returned in repeated fashion.

2. Description of the prior art
A well-known method of gathering blood plasma is to centrifugally separate whole blood taken from a healthy donor and collected in a blood bag. An apparatus has recently been developed for obtaining a large quantity of blood plasma from a single donor by means of semicontinuous or continuous centrifugation. Membrane-type blood plasma separators have also appeared and make it possible to separate large quantities of blood plasma anywhere with facility, and much research has been done in the area of so-called plasma exchange therapy, in which a large amount of blood plasma is separated from a patient' suffering from a serious immunological or metabolic illness and the patient is supplemented with blood plasma collected from a healthy donor. Blood plasma obtained from healthy donors is also used for such purposes as nutritional supply of gravity patients, supply of coagulation factor and invigoration of the immunity function, and demand for such plasma is ever increasing. For this reason, a variety of research is being conducted into methods of obtaining large quantities of blood plasma from healthy donors safely, inexpensively and with facility. The membrane-type plasma separator is particularly promising as it features a higher separating speed than a continuous centrifuge and can be handled without costly specialized equipment.

However, with the conventional continuous separation of blood plasma using a membrane, it is necessary for a vein to be punctured at two locations, namely, on the side at which blood is collected from e.g., a donor, and on the side at which blood is returned to the donor, and it is also required that continuous extracorporeal circulation of blood be performed. Accordingly, the risk of injury to the donor is high. Moreover, since a plasma pump is provided to prevent hemolysis due to a rise in TMP caused by resistance on the blood-return side, the separator operates at less than maximum performance. This results in a low separation efficiency.

With the conventional semicontinuous separation of blood plasma using a membrane, separator resistance at return of the blood from a blood reservoir rises owing to the concentrated blood obtained from the separation process. The result is damage to the blood cells and an extended period of time for return of the blood. Though a bypass can be provided to alleviate the problem, this would result in loss time equivalent to the time needed for the bypass. Furthermore, since the TMP internally of the separator in a one-pump system becomes a negative pressure at return of the blood so that the collected plasma flows backward, it is necessary to interrupt the separation process at return of the blood or to provide a blood return pump and a switching valve.

An apparatus according to the preamble part of Claims 1 and 6 is known from EP—A—0 085 016. This apparatus is adapted to sample blood from and to return blood to a donor via a single needle. Further, this apparatus includes a plasma separator for concentrating the blood and a reservoir for temporarily pooling concentrated blood. So, with the known apparatus the blood passes twice to separator, the first time when blood is collected in the reservoir and a second time when the blood is returned to the donor via the plasma separator. However, in this known apparatus, all the blood concentrated by the plasma separator is collected in the reservoir, and it is only returned to the plasma separator when the blood from the reservoir is returned to the donor. So, the known apparatus cannot achieve a plasma separation with a good efficiency.

In EP—A—0 131 529, which is based on a prior application, but has not been published before the priority date of this application, a similar apparatus is described comprising additionally an internal circulating circuit. In this apparatus according to the prior art, the efficiency of the plasma separation is improved. However, there is another disadvantage. When the blood circulating in the plasma separator is concentrated, a rise in viscosity results whenever plasma is separated from the blood. As a result, it becomes progressively more difficult for the blood to flow internally of the separator, and so, there is an increasing danger of hemolysis occurring.

The present invention has been devised to solve the aforementioned problems of the prior art, and an object of the invention is to provide an apparatus for separating blood plasma, wherein the vein of a donor need be punctured at only one location, the risk of extracorporeal blood circulation can be avoided, and plasma can be separated continuously from blood collection to blood return, thereby enabling plasma to be collected at an efficiency higher than that heretofore obtainable with a continuous system regardless of adoption of a semicontinuous plasma separation method in which blood collection and return are performed intermittently.

According to the present invention, the foregoing object is attained in a first embodiment with the features of Claim 1 and in a second embodiment with the features of Claim 6.

According to the invention, the membrane-type blood plasma separating means is provided with means for varying the cross-sectional area of blood passages internally of the separating means.

Additionally, the blood reservoir means may comprise a sealed bag made of a flexible synthetic resin. Further, the blood feed means for introducing and discharging blood may comprise a roller pump capable of forward and reverse rotation. Preferably, the apparatus of the invention further includes at least two pumps, one for driving the blood feed means and the other for driving the circulating means.

Other features and advantages of the present invention will be apparent from the following description taken in conjunction with the accompanying drawings, in which like reference characters designate the same or similar parts throughout the figures thereof.

Brief description of the drawings

Figure 1(a) is a perspective view showing a blood plasma separating apparatus according to ths present invention;

Figure 1(b) is a circuit diagram of a blood plasma separating apparatus according to the present invention;

Figure 1(C) is an enlarged sectional view showing an embodiment of a blood plasma separator;

Figure 1(d) is an enlarged sectional view showing a pressing device operatively associated with the separator of Figure 1(C);

Figures 2(A) and 2(B) are block diagrams illustrating the operating principle of the blood plasma separating apparatus at collection and return of blood.

Figure 3 is a graph illustrating the relation between the amount of blood plasma separated and the operating time of the apparatus;

Figure 4 is a circuit diagram of an experiment using the plasma separating apparatus for practicing the plasma separating method of the illustrated embodiment;

Figure 5 is a circuit diagram of an experiment indicative of the conventional continuous-type blood plasma separating method;

Figures 6(A) and 6(B) are block diagrams illustrating the operating principle of a modification of the blood plasma separating apparatus for practicing the blood plasma separating method of the illustrated embodiment:

Figure 7 is a circuit diagram of a second embodiment of a blood plasma separating apparatus according to the present invention;

Figures 8(A) and 8(B) are block diagrams illustrating the operating principle of the blood plasma separating apparatus at collection and return of blood;

Figure 9 is a circuit diagram of an experiment indicating the second embodiment of the plasma separating apparatus according to the present invention;

Figure 10 is a circuit diagram of an experiment indicating the conventional continuous-type separation method; and

Figures 11(A), 11(B), 12(A) and 12(B) are block diagrams illustrating modifications of the second embodiment of the blood plasma separating apparatus according to the present invention.

Description of the preferred embodiments

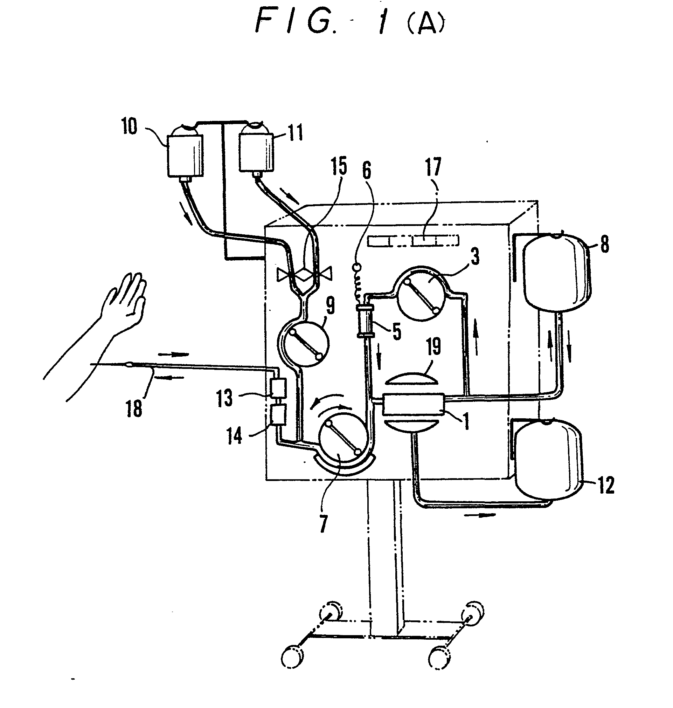

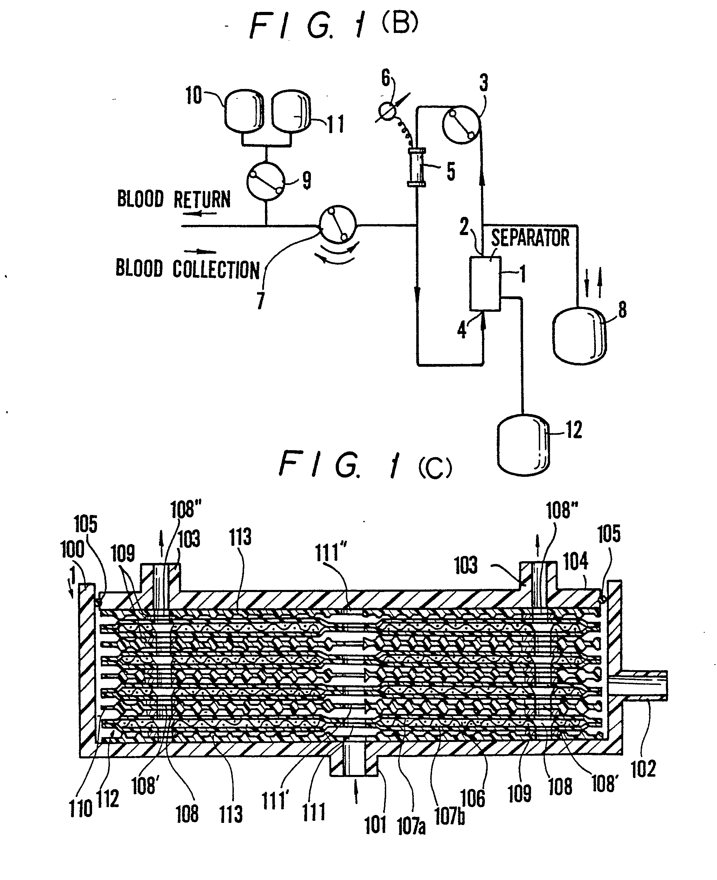

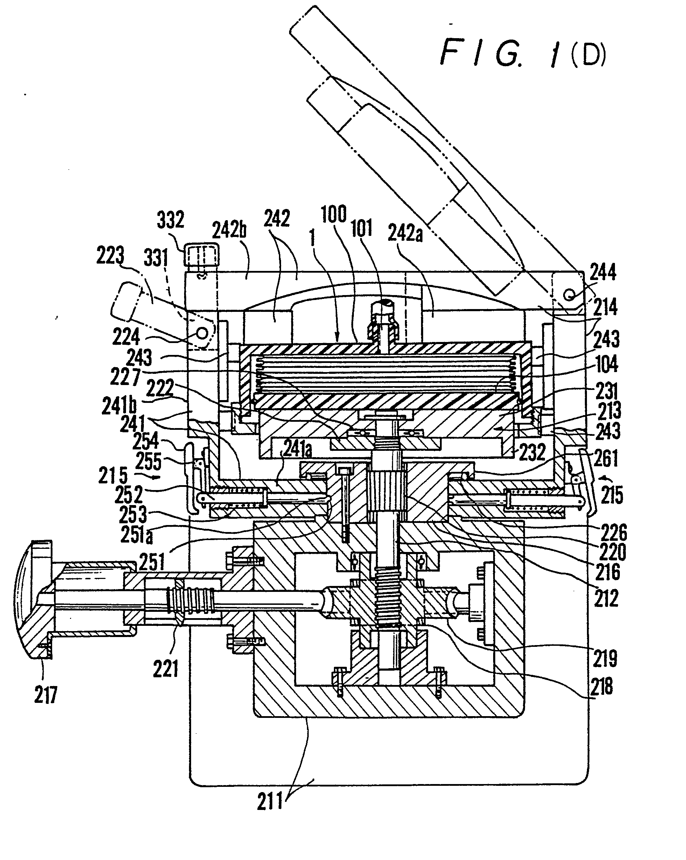

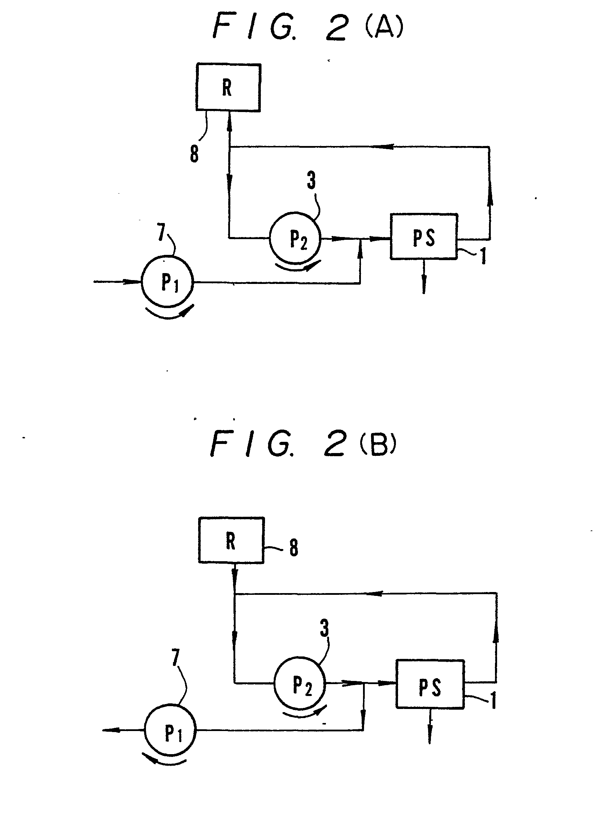

Figure 1(A) is a perspective view showing a blood plasma separating apparatus for practicing an embodiment of a blood plasma separating method, Figure 1(B) is a circuit diagram of the blood plasma separating apparatus shown in Figure 1(A), and Figures 2(A) and 2(B) are block diagrams illustrating the operating principle of the blood plasma separating apparatus at collection and return of blood.

With reference to Figure 1(A), the plasma separating apparatus of the present invention includes a membrane-type plasma separator 1, a recirculating pump 3 connected to the separator 1, a pressure monitoring air chamber 5 connected to the recirculating pump 3, a pressure gauge 6 connected to the air chamber 5, a blood feed pump 7 connected to the line between separator 1 and air chamber 5, a blood reservoir 8 connected to the line between the separator 1 and recirculating pump 3, a replacement liquid pump 9 connected to the blood feed pump 7, a vessel of anticoagulant 10 connected to the pump 9, a vessel of a replacement liquid 11 connected to the pump 9, and a plasma collecting vessel 12 connected to the separator 1. Also included are an air bubble detector 13, a negative pressure detector 14, a switching valve 15 for selecting either the anticoagulant 10 or the replacement liquid 11, a display and control section 17, a venipuncture needle 18, and a pressing device 19 serving as adjusting means for varying the cross-sectional area of blood flow passages in the separator 1.

As shown in the circuit diagram of Figure 1(B), the plasma separating apparatus separates plasma from blood by way of the membrane-type separator 1, includes a blood circulation circuit for recirculating the resulting concentrated blood from a blood outlet 2 of the plasma separator 1 to a blood inlet 4 thereof 1 by the recirculating pump 3, and causes blood collected from an individual, such as a donor or patient, to flow into the line of the circulation circuit between the recirculating pump 3 and the blood inlet 4 of plasma separator 1 by means of the blood feed pump 7, which is reversibly rotatable. Some of the concentrated blood from the blood outlet 2 of the plasma separator 1 branches from the circuit leading to the recirculating pump 3 and collects in the blood reservoir 8, with the amount of the blood fed to the plasma separator 1 being fixed. The arrangement is such that blood in the reservoir 8 and circulation circuit is returned to the individual by reversing the rotation of the blood feed pump 7. The pressure of the blood flowing into the plasma separator 1 of the recirculating circuit is monitored by the pressure gauge 6 connected to the pressure monitoring air chamber 5.

Let us now describe the operation of the plasma separating apparatus of the illustrated embodiment.

The interior of the circuitry is primed with a physiologic saline solution or the like introduced from the terminus of the circuitry by driving the pump 7. Following priming, the recirculating pump 3 is set into operation to form the blood circulating circuit, after which blood is introduced from the needle 18 at the terminus of the circuitry by rotating the pump 7 in the forward direction. At this time the anticoagualant 10, such as heparin, ACD solution or sodium citrate solution, should be mixed with the blood by operating the replacement liquid pump 9. The blood which has entered the recirculating circuit passes through the plasma separator 1, where plasma is separated from the blood and then collected in the plasma collecting vessel 12. Meanwhile, the concentrated blood resulting from the separation process in the plasma separator 1 is circulated through the circuit by the recirculating pump 3 to be diluted with blood obtained afresh from the donor and is reintroduced to the plasma separator 1 where further concentration is performed. Some of the concentrated blood which flows out of the separator 1 is pooled in the blood reservoir 8. It is preferred that the blood reservoir 8 comprise a sealed bag made of a flexible synthetic resin, for a reservoir of such construction will automatically follow up changes in pressure.

As soon as a predetermined amount of blood has been collected from the donor, the blood feed pump 7 is rotated in the reverse direction to cause the concentrated blood in reservoir 8 to flow back into the recirculating circuit. The concentrated blood thus enters the recirculating circuit, flows through the recirculating circuit and some of the concentrated blood is returned to the donor by the blood feed pump 7 while the remainder re-enters the plasma separator 1 from the blood inlet 4, whereby the blood is concentrated again. Some of this reconcentrated blood is returned to, e.g., the donor, by the blood feed pump 7. Plasma is continuously separated from the donor's blood by repeating this process. During this process the amount of the blood in the recirculating circuit must be larger than that of the blood to be returned to the donor. In other words, the amount of blood which is recirculated is the amount of blood flow produced by pump 3 minus the amount of blood flow produced by pump 7. At this time the diluting liquid 11, such a physiologic saline or glucose solution, may be administered to the donor, rather than the anticoagulant 10, by the pump 9. Note that a prescribed amount of blood will circulate through the membrane-type plasma separator 1 due to the inflow of blood to the reservoir 8 at blood collection and the outflow of blood from the reservoir 8 at blood return.

It is preferred that a separator of the kind shown in the enlarged sectional view of Figure 1(C) be used as the plasma separator 1. The structure of this separator will now be described.

The separator 1 comprises a case composed of a cylindrical case body 100 and a plunger 104, which serves also as the separator cap. The case body 100 has a bottom centrally provided with a body fluid (blood) inflow port 101, and a side wall provided with a filtration residue (concentrated blood) outflow port 102. The plunger 104 has filtrate (blood plasma) outflow ports 103, 103, and has an O-ring 105 attached to its outer circumferential portion. Disposed within the case are a plurality of filtration membrane units 112. Each unit 112 includes two circularly shaped filtering membranas 107a, 107b which embrace, from above and below, a filtrate flow passage forming plate 106 of circular shape comprising a screen mesh having a central opening 111 and filtrate passageways 108 located near the periphery of the plate. Each filtration membrane unit 112 has its outer circumferential portion as well as its inner circumferential portion about the central opening sealed as by thermal fusion or adhesive, and a sealing member 109 is bonded to the outer periphery of each filtrate passageway 108. It should be noted that the filtrate flow passage forming plate 106 is not limited to the above mentioned mesh; what is essential is that flow passages for the filtrate be assured.

Arranged between neighboring ones of the filtration membrane units 112 is a body fluid flow passage regulating plate 110 of circular shape having a central opening 111' and filtrate passageways 108' corresponding to those in the units 112, as well as a multiplicity of projections on the upper and lower surfaces thereof. Note that the outer peripheral portion of each filtrate passageway 108' has a flat configuration. Also provided in the case body 100 of the separator 1 are two body fluid flow passage regulating plates 113 each of circular shape having a multiplicity of projections on one side surface thereof. One of the plates 113 is arranged with its projections in abutting contact with the upper surface of the uppermost filtration membrane unit 112, and the other of the plates 113 is arranged with its projections in abutting contact with the lower surface of the lowermost filtration membrane unit 112. Each of the body fluid flow passage regulating plates 113 is provided with a central opening 111'' and filtrate passageways 108'' corresponding to those in the units 112. Note that the outer peripheral portion of each filtrate passageway 108'' has a flat configuration. The filtration membrane units 112 and the body fluid flow passage regulating plates 110, 113 are stacked in the manner described and inserted in the case body 100, the entirety is capped under pressure by the plunger 104 to fit the assembly snugly into the case body 100, and the O-ring 150 provides a liquid-tight seal between the plunger 104 and the case 100. The assembly that results is the plasma separator 1. Owing to the pressure applied by the plunger 104, the filtration membrane units 112 and the body fluid flow passage regulating plates 110, 113 are integrally joined at the outer peripheral portions of the filtrate passageways 108, 108', 108'' by means of the sealing members 109, so that these passageways are brought into communication with one another. It should be noted that Figure 1(C) depicts membrane thickness, the size of the projections and the spacing between the layers in exaggerated form.

Figure 1(D) is an enlarged sectional view showing the details of the pressing device 19 [Figure 1(A)]. A base 211 of suitable shape is provided with a pressing rod 212 capable of moving freely in the axial direction. The distal end of the pressing rod 212 is provided with a pressing plate 213 capable of being rotated freely about the axis of the pressing rod. Attached to the base 211 so as to rotate coaxially with respect to the pressing plate 213 is a frame-shaped holder 214 capable of accommodating the separator 1 in such a manner that the plunger 104 of the separator 1 may be abutted against the pressing plate 213. The holder 214 is provided with holder locking mechanisms 215 so that the holder can be fixed to the base 211 at a predetermined rotational position with respect thereto.

The arrangement for supporting the plasma separator 1 is thus characterized in that the pressing plate 213 is not only movable in unison with the pressing rod 212 in the axial direction thereof but is also freely rotatable about the axis of the pressing rod, the holder 214 is not only movable axially of the pressing rod 212 but is also rotatable coaxially with respect to the pressing plate 213, and the holder locking mechanisms 215 enable the holder 214 to be fixed to the base 211 at a predetermined rotational position.

The base 211 may consist of any suitable material and have any appropriate shape. As an example, the base 211 may have a columnar configuration and consist of cast iron. The pressing rod 212 is mounted on the base 211 so as to project therefrom and is freely movable axially of the plunger 104 held by the holder 214. In the illustrated embodiment, the pressing rod 212 is supported by the base 211 and by a bearing block 216 attached to the base 211 and, by turning a knob 217, may be moved freely in the axial direction so as to deliver a large axial thrust. A mechanism for rectilinear motion is interposed between the knob 217 and the pressing rod 212 and serves to convert horizontal motion of the knob 217 into axial motion of the rod 212. The mechanism may be of any suitable construction but in the illustrated embodiment includes a worm screw 218 coupled to the knob 217, and a worm 219 meshing with the worm screw 218. The pressing rod 212 is threadedly engaged with female screws provided on the worm 219 and is supported by the bearing block 216 (essentially the base 211) via a sliding key or spline 220, which prevents the pressing rod 212 from rotating. Turning the knob 217 rotates the worm 219 and the worm screw 218 meshing therewith, with the worm 219 rotating while in threaded engagement with the pressing rod 212. Since the pressing rod 212 is non-rotatable, it is free to move in the axial direction.

It should be noted that the bearing block 216 is essentially a part of the base 211. The amount by which the knob 217 is rotated is indicated by an indicator 221.

The pressing plate 213 is provided at the projecting end of the pressing rod 212 and is rotatable about the axis of the rod and movable axially in unison with the rod so as to apply pressure to the plunger 104 of the plasma separator 1.

In the illustrated embodiment, the pressing plate 213 comprises a disk portion 231 and a skirt portion 232 contiguous with the disk portion and covering the outer circumference of the flange portion 261 of the bearing block 216. The pressing plate 213 is fitted securely on the pressing rod 212 in such a manner that the skirt portion 232 covers a bearing flange 222 screwed securely onto the pressing rod 212.

The holder 214 surrounding the pressing plate 213 is mounted on the base 211 so as to be capable of rotating coaxially with respect to the pressing rod 212. The holder 214 has a frame-like construction and comprises a frame portion 241 having a cavity at a position facing the pressing plate 213 for receiving the plasma separator 1, and a hatch portion 242 for closing the cavity of the frame portion 241. The holder 214 is adapted to rigidly hold the case body 100 of the separator 1 in such a manner that the opening to the separator vessel confronts the pressing plate 213.

In the illustrated embodiment, the frame portion 241 of the holder 214 comprises a front part 241a and a pair of side parts 241b, 241b. The hatch portion 242 comprises a pressing part 242a and an arm part 242b. The hatch portion 242 may be swung open and closed relative to the frame portion 241 and, when closed, may be locked in place by fixing means 223. The front part 241a is fitted rotatably over the outer circumferential surface of the bearing block 216 and is embraced by the base 211 and the flange 261 of the bearing block 216. The two side parts 241b, 241b extend in parallel with the axis of the pressing rod 212 from respective ends of the front part 241a so as to surround the pressing plate 213, and each has a receiving projections 243, 243 on which the separator 1 is placed from below. The arm part 242b of the hatch portion 242 is formed integrally with the pressing part 242a and extends across the two side parts 241b, 241b of the frame portion 241. One end of the arm part 242b is pivotally connected by a pin shaft 244 to one end of one side part 241b, so that the other end of the arm part 242b may be swung about the pivot point. A slit (not shown) is provided in the arm part 242b so as to extend from said other end thereof to a point part of the way along the pressing part 242a and arm part 242b. Fitting the fixing means 223 into the slit locks the hatch portion 242 to the front part 241a in the closed condition.

The fixing means 223 has one end 331 thereof pivotally connected by a pin 224 to the side part 241b, and is formed to include a locking portion 332 at its other end. With the fixing means 223 fitted into the slit formed in the hatch portion 242, the locking portion 332 comes into abutting contact with the side face on the openable end of the arm part 242b from the outside thereof to lock the hatch portion 242 in place. Preferably, and by way of example, the fixing means 223 has a hatch locking mechanism for maintaining the locking engagement between the openable end of the arm part 242b and the corresponding side part 241b.

The holder locking mechanism 215 has a locking recess 251, a locking projection 251a provided at one end of a pin body 252, a resilient member 253, and a locking release lever 254. The locking recess 251 is provided in the outer circumferential surface of the bearing block 216 (essentially in the base 211 at a position opposing the holder 214). The pin body 252 having the locking projection 251a, the resilient member 253 and the locking release lever 254 are provided in the holder 214. The pin body 252 is linked with the locking release lever 254, and the locking projection 251a at the one end of the pin body 252 is urged by the resilient means 253 into engagement with the locking recess 251 at a prescribed rotational position of the holder 214. The resilient member 253 may be any suitable resilient means, such as a spring.

The locking release lever 254 should be provided at a desired position and operable in the radial direction, namely in a direction perpendicular to the rotational axis of the holder 214. In the illustrated embodiment, the locking release lever 254 is provided on the outer surface of the side part 241b at a position in the proximity of the base 211 and is pivotally supported at an intermediate point by a pin shaft 255 in such a manner that both ends thereof are capable of being swung outwardly at right angles to the axis of the pressing rod.

The pin body 252, resilient member 253 and locking release lever 254 are provided at two diametrically opposed locations, namely at two locations 180° apart circumferentially of the holder 214. Accordingly, when the holder 214 is rotated through 180° to position the two side parts 241b thereof in a horizontal plane which contains the pressing rod 212, the holder 214 will be locked automatically to the base 211.

In order to assure rotation of the pressing plate 213 and holder 214, a thrust bearing 226 is provided between the bearing block 216 and the holder 214, and a thrust bearing 227 is mounted between the bearing flange 222 and the pressing plate 213.

In operation, either of the locking projections 251a of the holder locking mechanism 215 is mated with the locking recess 251 to lock the holder 214 to the base 211. Under these conditions, the hatch portion 242 of the holder 214 is swung open horizontally, the body fluid inlet port 101 of the separator 1 is pointed upward, and the separator 1 is placed in the holder 214 through the opening thereof with the plunger 104 being faced toward the pressing plate 213. This is followed by closing the hatch portion 242, fitting the fixing means 223 into the slit formed in the hatch portion 242, and locking the hatch portion 242 in the closed state by the hatch locking mechanism.

The plunger 104 is moved up and down by turning the knob 217 back and forth. Moving the plunger 104 up and down causes a change in the cross-sectional area of the blood flow passages in the plasma separator 1. The blood circulating in the plasma separator 1 is concentrated and, hence, experiences a rise in viscosity whenever plasma is separated from it. As a result, it becomes progressively more difficult for the blood to flow internally of the separator 1, the pressure loss rises and TMP increases. If unchecked, this can lead to hemolysis, namely damage to the red blood cells. Accordingly, if the plunger 104 is lowered by turning the knob 217, the cross-sectional area of the blood flow passages increases, thereby diminishing pressure loss internally of the separator 1 and regulating TMP to within a prescribed range, thus preventing hemolysis. The plasma separator 1 is thus capable of completely separating plasma from entrant blood.

With the plasma separating apparatus for practicing the plasma separating method of the illustrated embodiment, blood concentrated once and pooled in the blood reservoir 8 is recirculated through the recirculating circuit and concentrated again by the separator 1 at return of the blood to the individual, so that plasma is collected even when blood is returned. This enables separation to be performed continuously without interruption when blood is returned.

Let us describe this process in slightly more detail with reference to Figures 2(A) and 2(B). Figure 2(A) illustrates the blood collection cycle. Blood feed pump (P1) 7 is rotated in the forward direction to draw blood from the donor and feed the blood into the circulating circuit. Recirculating pump (P2) 3, which always rotates in the forward direction circulates the blood through the plasma separator (PS) 1, where plasma is separated and stored in the plasma collecting vessel, not shown in Figure 2(A). The concentrated blood resulting from this separation process exits from the plasma separator 1 and flows into the circulating circuit owing to the action of the pump 3. Some of the concentrated blood enters the blood reservoir (R) 8 and the remainder reenters the inlet side of the plasma separator 1 before mixing with fresh blood introduced from the donor by the feed pump 7 upstream of the separator. The mixture of concentrated blood and fresh blood is subjected to plasma separation by the plasma separator 1. The foregoing steps are then repeated.

Figure 2(b) shows the blood return cycle performed when a predetermined amount of blood has been introduced into the system. Here the blood feed pump 7 is operated in the reverse direction to return the blood cell fraction to the donor from the point between the recirculating pump 3 and the inlet side of the plasma separator 1. As mentioned above, however, the recirculating pump 3 continues rotating in the forward direction. Accordingly, the concentrated blood pooled in the blood reservoir 8 during the collection cycle is now withdrawn from the reservoir and introduced into the circulating circuit by the pump 3. Some of this concentrated blood is returned to the donor upstream of the plasma separator 1 by the action of the blood feed pump 7, as mentioned above, and the remainder enters the plasma separator 1 where it is again subjected to plasma separation and, hence, reconcentration. It will thus be understood that plasma separation takes place even while blood is being returned to the donor.

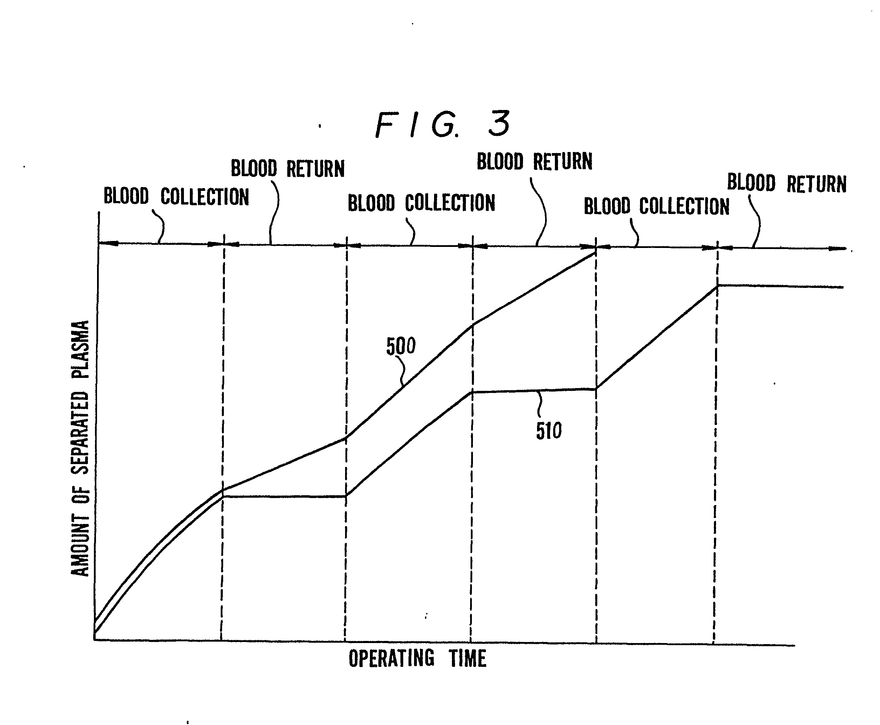

Figure 3 is a graph showing the relation between the amount of plasma separated and the operating time of the apparatus. Curve 510 represents the results achieved with a conventional semicontinuous membrane-type plasma separating apparatus, and curve 500 shows the results achieved with the apparatus of the illustrated embodiment. Curve 50 indicates that plasma separation takes place even during return of the blood and, hence, is carried out in a highly efficient manner. Curve 51 shows that since separation is suspended during return of the blood, a longer period of time is required to separate an equivalent amount of plasma with respect to curve 50.

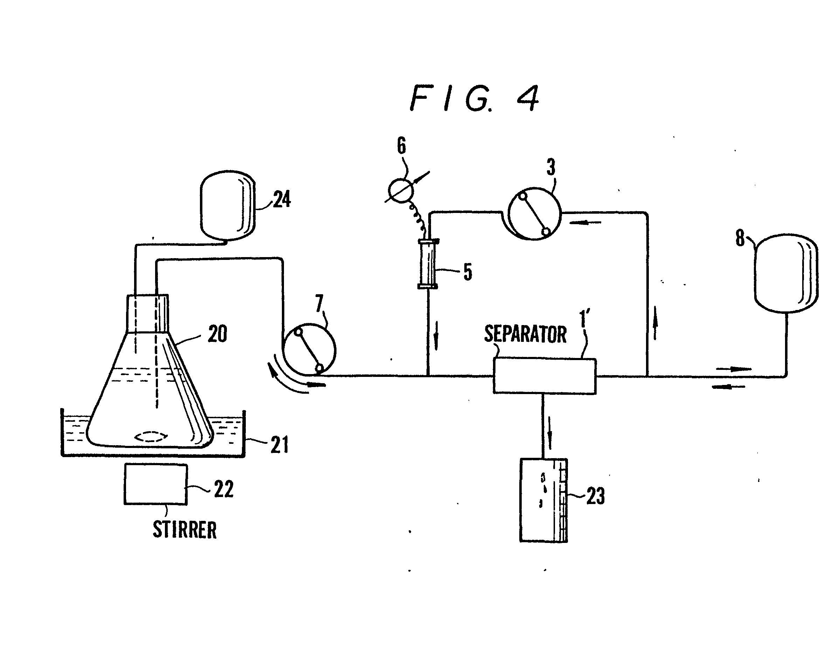

Figure 4 illustrates an example of an experimental set-up indicative of an embodiment of the plasma separating apparatus of the present invention. In the experiment, use was made of a membrane-type plasma separator 1' in which were stacked eight porous membranes each consisting of cellulose acetate with a pore size of 0.45 μm and having a donut-shaped configuration with an inner diameter of 3.6 cm and an outer diameter of 10 cm. The membranes presented an effective membrane surface area of 546.6 cm². The circuitry, inclusive of the separator 1', was arranged as shown. It should be noted that the membrane-type plasma separator is not limited to a flat membrane-type configuration, for a hollow fiber-type plasma separator can also be employed. Three liters of fresh bovine blood (hematocrit: 42%) with ACD serving as the anticoagulant was prepared in an Erlenmeyer flask 20 and the blood was stirred by a stirrer 22 in a water bath 21 at 37°C. The flask 20 containing the cow blood served as an imitation donor. After the interior of the circuitry was filled with a physiologic saline solution, the blood feed pump 7 was rotated to provide a blood flow rate $(Q_B)$ of 60 ml/min. At the same time, the recirculating pump 3 was rotated to provide a blood flow rate $(Q_R)$ of 110 ml/min. Plasma separation began immediately, with the separated plasma being received by a measuring cylinder 23 to measure the total amount (V) of plasma accumulated. Concurrently, blood inflow pressure (P) was measured for a requisite period of time by the pressure gauge 6 connected to the air chamber 5, with 0 min being taken as the start of blood extraction. When the total amount of blood extracted attained a value of 500 ml, the blood feed pump 7 was reversed to provide a return flow rate of 60 ml/min, and the recirculating pump 3 was rotated at a higher speed to increase the blood flow rate to 160 ml/min, thereby returning the blood to the flask 20. At the end of blood return, the Erlenmeyer flask 20 was replenished with an amount of physiologic saline 24 equivalent to the amount of plasma collected in the measuring cylinder 23. The foregoing process was repeated to provide the experimental results shown in the following Table 1:

TABLE 1

| Time (min) | Pressure (mmHg) P | Collection/ return flow rate (ml/min) $Q_B$ | Recirc. flow rate (ml/min) $Q_R$ | Amt. of separated plasma (ml) V |
|---|---|---|---|---|
| 0 | 75 | +60 | 110 | 0 |
| 2 | 100 | +60 | 110 | 60 |
| 4 | 135 | +60 | 110 | 120 |
| 6 | 140 | +60 | 110 | 175 |
| 8 | 145 | −60 | 200 | 230 |
| 10 | 140 | −60 | 200 | 250 |
| 12 | 145 | +60 | 110 | 270 |
| 14 | 140 | +60 | 110 | 325 |
| 16 | 145 | +60 | 110 | 375 |
| 18 | 150 | +60 | 110 | 425 |
| 20 | 135 | −60 | 200 | 445 |
| 22 | 140 | −60 | 200 | 460 |
| 24 | 120 | +60 | 110 | 485 |
| 26 | 135 | +60 | 110 | 515 |
| 28 | 140 | −60 | 200 | 525 |
| 30 | — | — | — | — |

In Table 1, the plus signs in the collection/return flow rate column indicate blood collection and the minus signs indicate blood return.

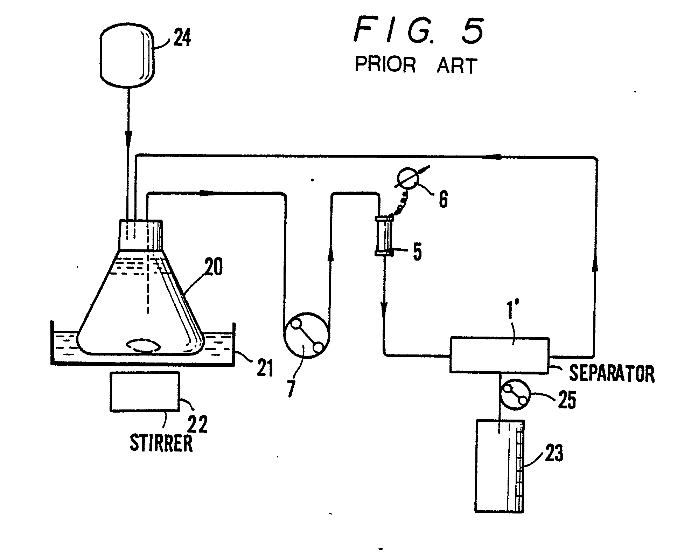

For the purpose of comparison, the inventor performed a plasma separation experiment using 3 l of fresh cow blood mixed with ACD, as in the experiment of Figure 4, employing a continuous separation method considered to be the most efficient at the present time. The experimental set-up, shown in Figure 5, utilizes the plasma separator 1', which has the same specifications at the separator 1' employed in the experiment of Figure 4. The results obtained are as shown in Table 2.

TABLE 2

| Time (min) | Pressure (mmHg) | Collection/ return flow rate (ml/min) | Amount of separated plasma (ml)· |
|---|---|---|---|
| 0 | 60 | 60 | 0 |
| 2 | 135 | 60 | 40 |
| 4 | 140 | 60 | 75 |
| 6 | 145 | 60 | 110 |
| 8 | 145 | 60 | 145 |
| 10 | 145 | 60 | 180 |
| 12 | 145 | 60 | 215 |
| 14 | 145 | 60 | 250 |
| 16 | 145 | 60 | 280 |
| 18 | 145 | 60 | 310 |
| 20 | 145 | 60 | 340 |
| 22 | 150 | 60 | 370 |
| 24 | 145 | 60 | 405 |
| 26 | 145 | 60 | 435 |
| 28 | 145 | 60 | 470 |
| 30 | 145 | — | 500 |

In the experiments of Figures 4 and 5, the cross-sectional area of blood flow passages in the separator 1 was so controlled by the pressing device as to keep the pressure from exceeding 150 mmHg.

A comparison of Tables 1 and 2 shows that 500 ml of plasma can be collected in less than 26 min with the separating method of the illustrated embodiment, while it takes 30 min to collect 500 ml of plasma with the continuous separation method of Figure 5. It will be appreciated that plasma can be separated from blood in a highly efficient manner according to the illustrated embodiment.

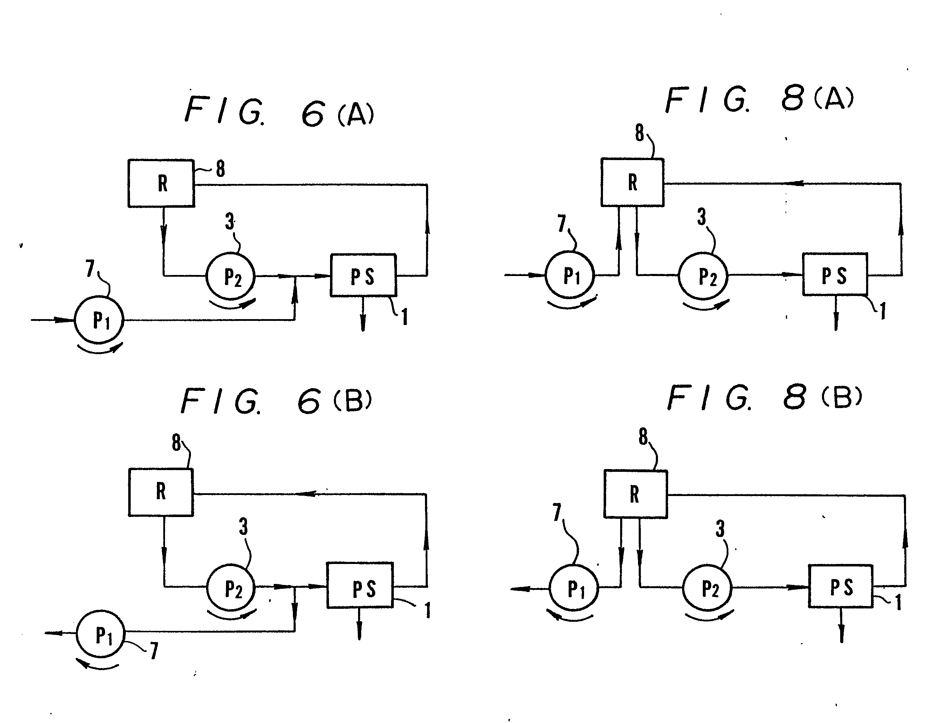

Figures 6(A) and 6(b) are block diagrams of a modification of the invention. In the illustrated arrangement, the reservoir 8 is incorporated into the recirculating circuit. Figure 6(A) illustrates operation at blood collection, and Figure 6(B) shows operation at blood return. The results obtained are the same as those with the arrangement of Figures 2(A) and 2(B). However, with the set-up shown in Figures 2(A), 2(B), the concentrated blood finds refuge in the blood reservoir 8 so that the concentration of the blood inside the reservoir 8 is substantially constant. With the set of shown in Figures 6(A), 6(B), on the other hand, blood in the reservoir 8 is being continuously circulated and concentrated at all times so that the blood concentration internally of the reservoir changes.

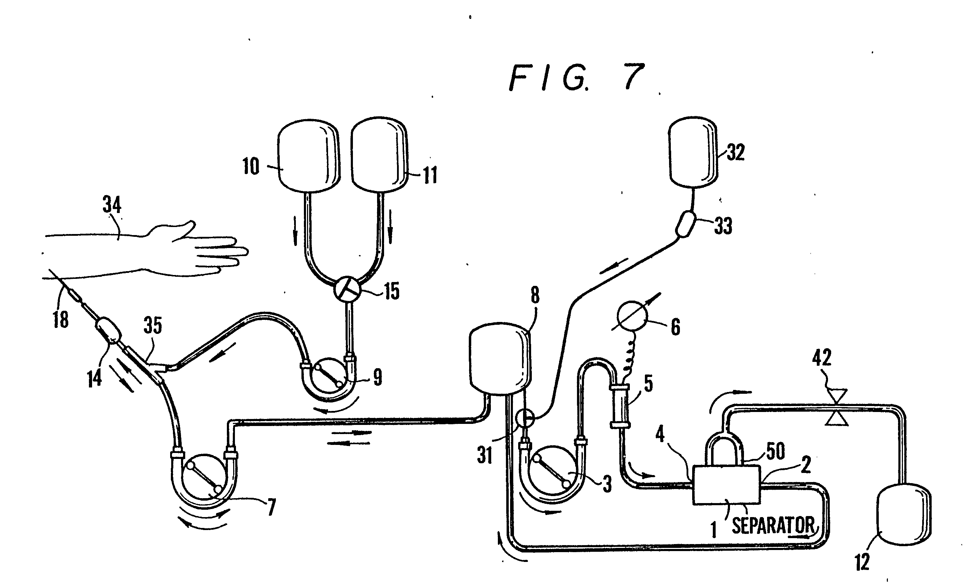

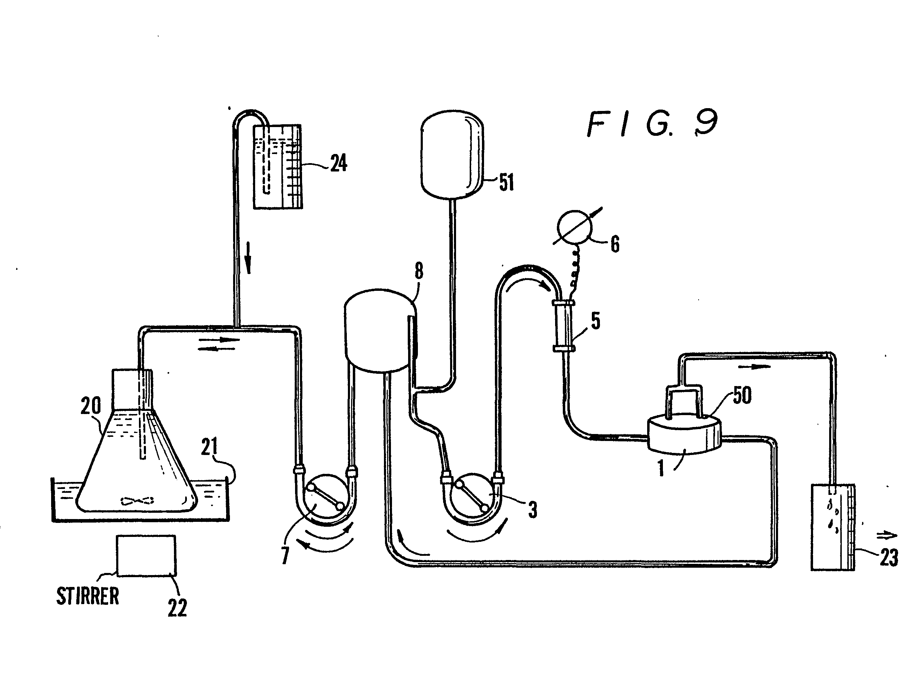

A second embodiment of the present invention will now be described. Figure 7 is a circuit diagram of a blood plasma separating apparatus of the second embodiment according to the present invention and Figures 8(A) and 8(B) are block diagrams illustrating the operating principle of the blood plasma separating apparatus at collection and return of blood for practicing the second embodiment of the plasma separation method. Portions similar to those shown in Figures 1(A) and 1(B) are designated by like reference characters. The second embodiment of the apparatus includes a three-way valve 31 in the line between the recirculating pump 3 and the blood reservoir 8, a vessel of physiologic saline solution 32, and a transfusion solution set 33 connected between the three-way valve 31 and the physiological saline solution vessel 32. Numeral 34 denotes the forearm of a donor. Unlike the first embodiment, blood from·the donor is introduced directly to the blood reservoir 8, rather than being fed directly into the blood circulating circuit.

In the operation of the second embodiment of the present invention, the three-way valve 31 is opened and the pump 3 is set into operation to fill the circuitry with the physiologic saline solution 32 introduced from the transfusion solution set 33. Next, the blood feed pump 7 is driven into rotation to collect blood from the needle 18 inserted into a vein in the donor's forearm 34. The negative pressure monitor 14 for monitoring the state of blood collection from the donor and a brancher 35 can be provided in the tubing between the needle 18 and the blood feed pump 7, and the anticoagulant 10 or physiological saline 11 can be mixed with the blood by changing over the valve 15 and driving the pump 9 at a rate fixed in proportion to the operating rate of the blood feed pump 7. Whole blood collected by operation of the feed pump 7 begins to pool in the blood reservoir 8, which is made of a flexible synthetic resin. At the same time, the recirculating pump 3 is operated so that the blood pooled in the reservoir 8 flows into the plasma separator 1 from the blood inlet 4 thereof via the air chamber 5. Plasma separated inside the separator 1 flows from a filtrate outflow port 50 thereof into the tubing of the recirculating circuit for collection in the plasma collecting vessel (collecting bag) 12 by opening a valve 42.

The concentrated blood in the separator 1 resulting from separation of the plasma flows out from the blood outlet 2, returns to the reservoir 8 where mixing with newly collected blood takes place, and is then fed again, together with the fresh blood, from the reservoir 8 into the separator 1 by the pump 3. Repeating this process enables plasma to be separated from blood in continuous fashion. The concentration of the blood in the blood reservoir 8 is monitored by the pressure gauge 6.

When collection of a predetermined amount of blood from the donor by operation of the blood fed pump 7 ends, the valve 15 is switched so that the donor may be administered replacement physiological saline or glucose 11 in an amount equivalent to the amount of separated plasma by operation of the pump 9. At the same time, the blood feed pump 7 is reversed to return the blood in reservoir 8 to the donor. The recirculating pump 3 continues operating even during return of the blood, so that the plasma separator 1 goes on concentrating the blood up to the final target concentration. As soon as the concentrated blood have been returned to-the donor, the blood feed pump 7 is again rotated in the forward direction to start collecting fresh blood from the donor so that the foregoing process may be repeated.

Thus, as in the first embodiment of the present invention, the above-described second embodiment also causes concentrated blood in the reservoir 8 to be subjected to concentration again by the separator 1 when blood is returned, so that plasma is separated from the concentrated blood even during the return stage of the process. Accordingly, the relation between the amount of plasma separation and the apparatus operating time is similar to that shown in the graph of Figure 3, so that approximately the same effect is obtained and a highly efficient blood collecting operation made possible.

Described next will be an experiment indicative of the plasma separating method of the second embodiment. The set-up is as shown in Figure 9.

Three liters of fresh bovine blood (hematocrit: 42%) with ACD serving as the anticoagulant was prepared in an Erlenmeyer flask 20 and the blood was maintained at a temperature of 37°C by a constant temperature bath 21. At the same time, the blood in the flask 20 was stirred by a stirrer 22 so that the blood would separate by standing. Next, the pump 3 was set into operation to fill the circuitry with the minimum amount of the necessary physiologic saline solution 51. The blood feed pump 7 was rotated to provide a blood flow rate of 60 ml/min and to introduce the blood into the blood reservoir 8.

At the same time, the recirculating pump 3 was rotated to provide a blood flow rate of 150 ml/min, introduce the blood through the air chamber 5 into the membrane-type separator 1', and then return the blood from the separator 1' to the reservoir 8, thereby setting up a circulating flow. At the same time that this closed circuit became filled with blood, the filtrate outflow port 50 was opened to collect the separated plasma in the measuring cylinder 23. The pressure of the blood fed into the separator 1' by the pump 3 was monitored by the pressure gauge 6 connected to the air chamber 5. The membrane-type plasma separator 1' was composed of eight stacked flat-type porous membranes each consisting of cellulose acetate with a pore size of 0.45 μm and having a donut-shaped configuration with an inner diameter of 3.6 cm and an outer diameter of 10 cm. The membranes presented an effective membrane surface area of 546.6 cm². The plasma separator 1' was filled with 12 ml of blood. In order to hold the filtration pressure constant, the structure of the separator 1' was such that the cross-sectional area of the blood flow passages could be varied in accordance with the concentration of the blood in the reservoir 8 as monitored by the pressure gauge 6. Though not employed, a hollow fiber-type membrane configuration can be adapted to the separator 1'. The results of the experiment are as shown in Table 3.

TABLE 3

| Operating time (min) | Flow rate | | P (mmHg) | V (ml) |
|---|---|---|---|---|
| | QB1 (ml/min) | QB2 (ml/min) | | |
| 0 | +60 | 0 | 0 | 0 |
| 2 | +60 | 150 | 78 | — |
| 3 | +60 | 160 | 100 | 50 |
| 6 | +60 | 160 | 140 | 140 |
| 8 | +60 | 160 | 150 | 200 |
| 9 | −60 | 160 | 150 | 230 |
| 12 | +60 | 160 | 165 | 255 |
| 14 | +60 | 160 | 150 | 290 |
| 18 | +60 | 160 | 155 | 350 |
| 21 | −60 | 160 | 157 | 400 |
| 27 | +60 | 160 | 160 | 440 |
| 28 | −60 | 160 | 160 | 475 |
| 30 | +60 | 160 | 154 | 485 |
| 31 | −60 | 160 | 160 | 500 |
| 33 | −60 | 160 | 0 | — |

The operating time column represents elapsed time immediately after start of blood collection. For example, the table shows that 500 ml of plasma were separated in 31 min. QB1 denotes the blood feed rate of the blood feed pump 7, where the plus sign indicates blood collection and the minus sign blood return. QB2 designates blood flow rate performed by the recirculating pump 3. P indicates that the blood flow passage pressure is controlled and held at a substantially constant value by monitoring the pressure at the inlet to the plasma separator 1' by the pressure gauge 6. V represents the total amount of plasma separated and shows that separation is carried out even when blood is returned to the donor. At return of the blood, physiologic saline 24 in an amount equivalent to the separated amount of plasma was returned to the flask 20. The maximum amount of blood collected in the reservoir 8 from the flask 20 was 500 ml.

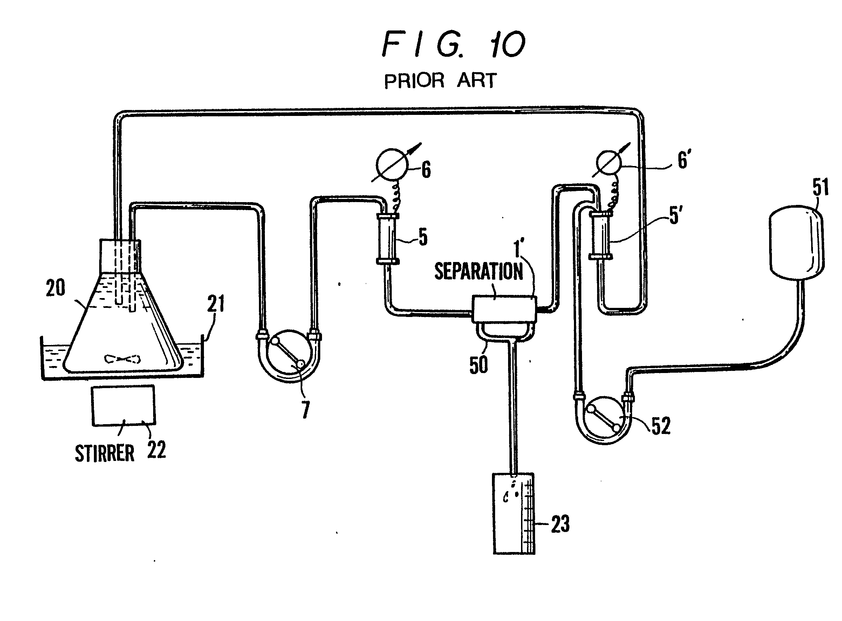

For the purpose of comparison, the inventor performed a plasma separation experiment using 3 l of fresh bovine blood (hematocrit: 40%) mixed with ACD and employing the membrane-type plasma separator 1' of Figure 9. As shown in Figure 10, the plasma was separated by the conventional continuous process. The bovine blood, prepared in the flask 20, was held at 37°C in the constant temperature bath 21 and was stirred to uniformity in the flask 20 by the stirrer 22. At a collecting flow rate of 60 ml/min, blood was fed into the separator 1' through the air chamber 5 by the blood feed pump 7, and the separated plasma was collected in the measuring cylinder 23 from the filtrate outflow port 50 of the separator 1'. The physiologic saline 51 in an amount equivalent to that of the separated plasma was delivered by a pump 52 to a second air chamber 5', where the saline solution was mixed with the concentrated blood. The mixture of physiologic saline and concentrated blood was returned to the flask 20. The pressures on the inflow and outflow sides of the separator 1' were monitored by pressure gauges 6, 6' connected to the respective chambers 5, 5', and the flow passage pressure internally of the separator 1' was regulated to give a pressure loss similar to that obtained in the above experiment. The experimental results are shown in Table 4.

TABLE 4

| Operating time (min) | Flow rate (ml/min) | Inflow pressure (mmHg) | Outflow pressure (mmHg) | Amount of separated plasma (ml) |
|---|---|---|---|---|
| 0 | 60 | 145 | 0 | — |
| 5 | 60 | 150 | 0 | 75 |
| 10 | 60 | 150 | 0 | 150 |
| 15 | 60 | 150 | 0 | 230 |
| 20 | 60 | 150 | 0 | 295 |
| 25 | 60 | 150 | 0 | 360 |
| 30 | 60 | 150 | 0 | 420 |
| 35 | 60 | 154 | 2 | 480 |
| 37 | 60 | 148 | 0 | 500 |
| 38 | 60 | 150 | 3 | — |

The operating time column represents elapsed time from the start of separation to the conclusion or blood return. Specifically, the table shows that 500 ml of plasma were obtained in 37 min and that 38 min was needed for the conclusion of the entire operation inclusive of blood return.

The blood flow rate is the flow rate established by the pump 7, the inflow and outflow pressures are those measured by the respective pressure gauges 6, 6', and the amount of separated blood plasma is the total accumulated amount measured by the measuring cylinder 23.

A comparison of Tables 3 and 4 clearly shows that the plasma separating apparatus for practicing the plasma separating method of the second embodiment separates plasma more efficiently than the conventional continuous separation method.

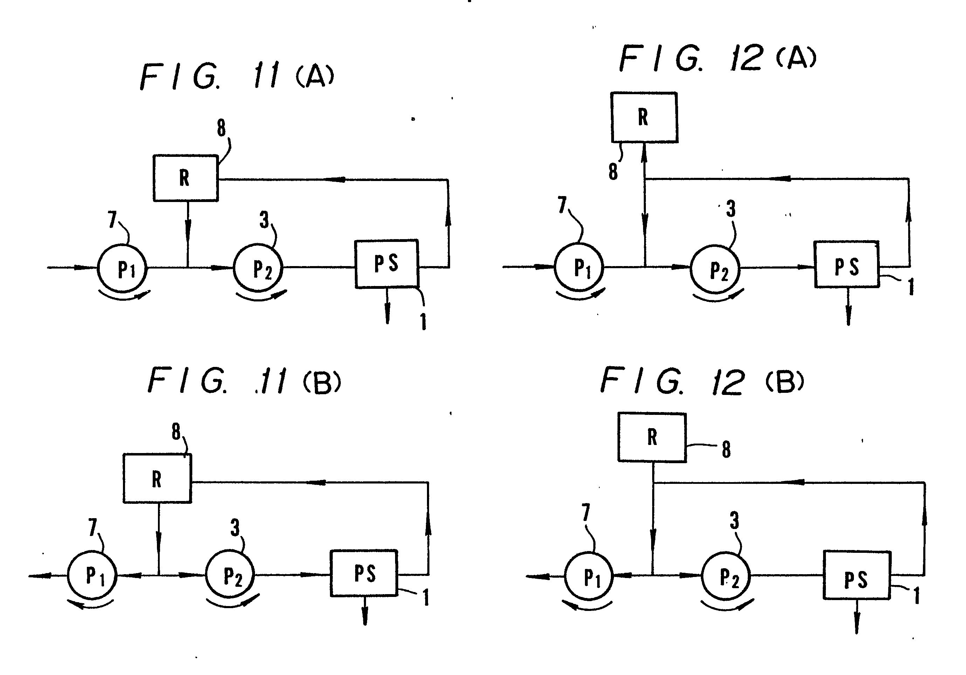

Figures 11(A) and 11(B) are block diagrams showing a modification of the second embodiment of Figures 7 and 8. Here the blood from the blood feed pump 7 does not flow directly into the blood reservoir 8. Figures 12(A) and 12(B) are block diagrams showing another modification of the second embodiment. Here the reservoir 8 is provided outside of the recirculating circuit, and the blood from the blood feed pump 7 does not flow directly into the blood reservoir 8. (A) in Figures 11 and 12 illustrates operation during blood collection, and (B) illustrates operation during blood return. The effects obtained with the arrangements of Figures 11 and 12 are similar to those obtained with the second embodiment shown in Figure 8. In the case of Figure 8, blood from the blood feed pump 7 and the concentrated blood from the plasma separator 1 mix in the blood reservoir 8, whereas in Figures 11 and 12 only the concentrated blood from the plasma separator 1 flows into the reservoir 8.

As many apparently widely different embodiments of the present invention can be made without departing from the spirit and scope thereof, it is to be understood that the invention is not limited to the specific embodiments thereof except as defined in the appended claims.

Concrete effects of the invention

According to the present invention as set forth above, plasma is separated from blood through a semicontinuous apparatus whereby a fixed amount of blood is repeatedly collected in and returned from a blood reservoir without requiring continuous circulation externally of a donor or patient. Therefore, unlike the conventional continuous arrangement that requires the donor or patient to be punctured at two locations, puncture at only one location is sufficient. Thus, a healthy donor is not subjected to the hazards of external circulation. Moreover, since plasma separation continues even during return of the blood and there is no need to operate the separator at less than peak performance as in the continuous system of the prior art, an equivalent or higher separation efficiency can be obtained, with one and the same plasma separator, as compared with the conventional external circulation-type continuous separation system heretofore considered to be the most outstanding in terms of effectiveness. The present invention therefore enables blood to be collected in a shorter period of time.

In the plasma separating apparatus of the present invention, the membrane-type plasma separating means is capable of having the cross-sectional area of its blood flow passages varied. This makes it possible to prevent hemolysis by suppressing a rise in pressure loss and stabilizing TMP even when blood viscosity changes. Since the blood reservoir means may comprise a sealed bag made of flexible synthetic

# EP 0 171 749 B1

resin, changes in pressure can be followed up automatically. Further, since the blood feed means for introducing and discharging blood may comprise a reversible roller pump, the cost of the apparatus can be lowered since only a single such roller pump need be used.

The plasma separating apparatus of the invention has at least two pumps, one for driving the blood feed means and the other for driving the circulating means. This enables a higher plasma separation efficiency to be achieved because the introduction, discharge and circulation of blood can be performed simultaneously.

## Claims

1. An apparatus for separating plasma from blood comprising:

a single common needle (18) for introducing blood and returning blood, membrane-type plasma separating means (1) having an inlet port (4) for introducing blood therein and an outlet port (2) for discharging concentrated blood therefrom,

blood feed means which includes a reversibly rotatable blood feed pump (7) for introducing and discharging blood from said single common needle, and

blood reservoir means (8) connected to said plasma separating means for temporarily pooling or collecting concentrated blood, characterized in that

a blood circulating circuit is provided having one end which is connected to said inlet port (4) of said plasma separating means (1) and another end connected to said outlet port (2) of said plasma separating means (1), the blood concentrated by said plasma separating means (1) flowing through said blood circulating circuit;

circulating means (3) is provided for circulating the blood internally of said blood circulating circuit from said outlet port (2) to said inlet port (4) of said plasma separating means (1);

said blood reservoir means (8) is coupled to said blood circulating circuit on an outlet side thereof for temporarily pooling or collecting an excess amount of concentrated blood which overflows in said blood circulating circuit;

said blood feed means is coupled between said single common needle (18) and said blood circulating circuit; and

said plasma separating means (1) further includes means (19) for varying the cross-sectional area of blood flow passages defined therein.

2. The apparatus according to Claim 7, wherein said blood reservoir means (8) comprises a sealed bag made of a flexible synthetic resin.

3. The apparatus according to Claim 7, further comprising at least two pumps (7, 3), one (7) for driving said blood feed means and the other (3) for driving said circulating means.

4. The apparatus according to Claim 7, further comprising at least two liquid-containing vessels (10, 11), and liquid feed means operatively associated with said blood feed means (7) for selectively delivering to said blood feed means a liquid from said liquid-containing vessels at a predetermined ratio.

5. The apparatus according to Claim 12, wherein the liquid contained in one (10) of said liquid-containing vessels is an anticoagulant, and the liquid contained in the other (11) of said liquid containing vessels is a replacement liquid of an amount equivalent to an amount of separated plasma.

6. An apparatus for separating plasma from blood comprising:

a single common needle (18) for introducing blood and returning blood, membrane-type plasma separating means (1) having an inlet port (4) for introducing blood therein and an outlet port (2) for discharging concentrated blood therefrom,

blood feed means which includes a reversibly rotatable blood feed pump (7) for introducing and discharging blood from said single common needle, and

blood reservoir means (8) connected to said plasma separating means for temporarily pooling or collecting concentrated blood, characterized in that

a blood circulating circuit is provided having one end which is connected to said inlet port (4) of said plasma separating means (1) and another end which is connected to said outlet port (2) of said plasma separating means (1), the blood concentrated by said plasma separating means (1) flowing through said blood circulating circuit;

circulating means (3) is provided for circulating the blood internally of said blood circulating circuit from said outlet port (2) to said inlet port (4) of said separating means (1);

said blood reservoir means (8) is provided in said blood circulating circuit, for temporarily pooling or collecting blood introduced through said single common needle (18) and concentrated by said plasma separating means;

said blood feed means is coupled between said single common needle (18) and said blood reservoir means (8), and

said plasma separating means (1) further includes means for varying the cross-sectional area of blood flow passages defined therein.

7. The apparatus according to Claim 6, wherein said blood reservoir means (8) comprises a sealed bag made of a flexible synthetic resin.

12

8. The apparatus according to Claim 6, further comprising at least two pumps (7, 3), one for driving said blood feed means and the other for driving said circulating means.

9. The apparatus according to Claim 6, further comprising at least two liquid-containing vessels (10, 11), and liquid feed means operatively associated with said blood feed means for selectively delivering to said blood feed means a liquid from said liquid-containing vessels at a predetermined ratio.

10. The apparatus according to Claim 9, wherein the liquid contained in one (10) of said liquid-containing vessel is an anticoagulant, and the liquid contained in the other (11) of said liquid containing vessels is a replacement liquid of an amount equivalent to an amount of separated plasma.

**Patentansprüche**

1. Vorrichtung zum Abtrennen von Blutplasma von Blut, welche aufweist:

eine einzelne gemeinsame Nadel (18) zum Zuführen und zum Rückführen von Blut,

eine membranartige Plasma-Trennvorrichtung (1) mit einer Einlaßöffnung (4) zum Einbringen von Blut in diese und einer Auslaßöffnung (2) zum Austragen von konzentriertem Blut aus dieser,

eine Blutfördereinrichtung, welche eine reversibel drehbare Blutförderpumpe (7) zum Einführen und Austragen von Blut von der einzelnen gemeinsamen Nadel enthält, und

einen Blutvorratsbehälter (8), welcher mit der Plasma-Trenneinrichtung verbunden ist, zum zeitweisen Speichern oder Sammeln von konzentriertem Blut, dadurch gekennzeichnet,

daß ein Blutumwälzkreislauf vorgesehen ist, welcher mit einem Ende an die Einlaßöffnung (4) der Plasmatrenneinrichtung (1) und mit einem anderen Ende an die Auslaßöffnung (2) der Plasmatrenneinrichtung (1) angeschlossen ist, wobei das durch die Plasmatrenneinrichtung (1) konzentrierte Blut durch den Blutumwälzkreislauf fließt,

daß eine Umwälzeinrichtung (3) vorgesehen ist, um das Blut innerhalb des Blutumwälzkreislaufes von der Ausgangsöffnung (2) zu der Eingangsöffnung (4) der Plasmatrenneinrichtung (1) umzuwälzen,

daß der Blutvorratsbehälter (8) an den Blutumwälzkreislauf an dessen Auslaßseite angeschlossen ist, um einen Überschußbetrag von konzentriertem Blut, welcher in dem Blutumwälzkreislauf in Übermenge vorhanden ist, zeitweise zu speichern bzw. zu sammeln,

daß die Blutfördereinrichtung zwischen den einzelnen gemeinsame Nadel (18) und dem Blutumwälzkreislauf eingeschaltet ist und

daß die Plasmatrenneinrichtung (1) außerdem eine Einrichtung (19) zur Veränderung der Querschnittsfläche der in ihr definierten Blut-Strömungsdurchgänge aufweist.

2. Vorrichtung nach Anspruch 1, dadurch gekennzeichnet, daß der Blutvorratsbehälter (8) einen aus flexiblem Kunstharzmaterial bestehenden abgedichteten Beutel umfaßt.

3. Vorrichtung nach Anspruch 1, dadurch gekennzeichnet, daß sie mindestens zwei Pumpen (7, 3), die eine (7) zum Antrieb der Blutfördereinrichtung und die andere (3) zum Antrieb des Umwälzkreislaufes, aufweist.

4. Vorrichtung nach Anspruch 1, dadurch gekennzeichnet, daß mindestens zwei Flüssigkeit enthaltende Behälter (10, 11) und eine mit der Blutfördereinrichtung (7) wirkungsmäßig verbundene Flüssigkeitsfördereinrichtung vorgesehen sind, um wahlweise eine Flüssigkeit von dem Flüssigkeit enthaltenden Behälter in einem vorgegebenen Verhältnis zu der Blutfördereinrichtung zu liefern.

5. Vorrichtung nach Anspruch 12, dadurch gekennzeichnet, daß die in einem (10) der Flüssigkeit enthaltenden Behälter enthaltene Flüssigkeit ein Antigerinnungsmittel ist und daß die in dem anderen (11) der Flüssigkeit enthaltenden Behälter enthaltende Flüssigkeit eine Ersatzflüssigkeit in einem dem Betrag des abgetrennten Plasmas äquivalenten Betrag ist.

6. Vorrichtung zum Abtrennen von Blutplasma von Blut, welche aufweist:

eine einzelne gemeinsame Nadel (18) zum Zuführen und Rückführen von Blut,

eine membranartige Plasmatrenneinrichtung (1) mit einer Einlaßöffnung (4) zum Einführen von Blut in diese und einer Auslaßöffnung (2) zum Austragen von konzentriertem Blut aus dieser,

eine Blutfördereinrichtung, welche eine reversibel drehbare Blutförderpumpe (7) zum Zuführen und Austragen von Blut von der einzelnen gemeinsamen Nadel umfaßt, und

eine Blutbehältereinrichtung (8), welche mit der Plasmatrenneinrichtung verbunden ist, zum zeitweiligen Speichern bzw. Sammeln von konzentriertem Blut, dadurch gekennzeichnet,

daß ein Blutumwälzkreislauf vorgesehen ist, welcher mit einem Ende an die Einlaßöffnung (4) der Plasmatrenneinrichtung (1) und mit einem anderen Ende an die Auslaßöffnung (2) der Plasmatrenneinrichtung angeschlossen ist, wobei das durch die Plasmatrenneinrichtung (1) konzentrierte Blut durch den Blutumwälzkreislauf fließt,

daß eine Umwälzeinrichtung (3) zum Umwälzen des Blutes innerhalb des Blutumwälzkreislaufes von der Auslaßöffnung (2) zu der Einlaßöffnung (4) der Plasmatrenneinrichtung (1) vorgesehen ist,

daß die Blutvorratsbehältereinrichtung (8) in dem Blutumwälzkreislauf vorgesehen ist, um zeitweilig durch die einzelne gemeinsame Nadel (18) eingeführtes und durch die Plasmatrenneinrichtung konzentriertes Blut zu speichern bzw. zu sammeln,

daß die Blutfördereinrichtung zwischen die einzelne gemeinsame Nadel (18) und die Blutvorratsbehältereinrichtung (8) eingeschaltet ist und

daß die Plasmatrenneinrichtung (1) außerdem eine Einrichtung zur Veränderung der Querschnittsfläche der in ihr definieren Blutströmungsdurchgänge aufweist.

7. Vorrichtung nach Anspruch 6, dadurch gekennzeichnet, daß die Blutvorratsbehältereinrichtung (8) einen aus flexiblem Kunstharz bestehenden, verseigelten Beutel aufweist.

8. Vorrichtung nach Anspruch 6, dadurch gekennzeichnet, daß mindestens zwei Pumpen (7, 3), die eine zum Antrieb der Blutfördereinrichtung und die andere zum Antrieb der Umwälzeinrichtung, vorgesehen sind.

9. Vorrichtung nach Anspruch 6, dadurch gekennzeichnet, daß sie mindestens zwei Flüssigkeit enthaltende Behälter (10, 11) sowie eine mit der Blutfördereinrichtung wirkungsmäßig verbundene Flüssigkeitsfördereinrichtung zur wahlweisen Lieferung einer Flüssigkeit von den Flüssigkeit enthaltenden Behältern in einem vorgegebenen Verhältnis an die Blutfördereinrichtung aufweist.

10. Vorrichtung nach Anspruch 9, dadurch gekennzeichnet, daß die in einem (10) der Flüssigkeit enthaltenden Behälter enthaltene Flüssigkeit ein Antigerinnungsmittel ist und daß die in dem anderen (11) der Flüssigkeit enthaltenden Behälter enthaltene Flüssigkeit eine Ersatzflüssigkeit in einem dem Betrag des abgetrennten Plasmas äquivalenten Betrag ist.

## Revendications

1. Appareil pour séparer le plasma du sang comprenant:
une aiguille commune (18) unique destinée à l'introduction du sang et au retour du sang, un moyen de séparation de plasma (1) du type membrane comportant un orifice d'entrée (4) pour y introduire le sang et un orifice de sortie (2) pour en évacuer le sang concentré, ‘
un moyen de transport de sang qui comporte une pompe de transport (7) pour le sang susceptible de tourner de façon réversible, destinée à l'introduction du sang et à l'évacuation du sang par ladite unique aiguille commune et un moyen (8) formant réservoir pour le sang, relié audit moyen de séparation de plasma pour accumuler ou recueillir temporairement le sang concentré, caracterise en ce que:
un circuit pour la circulation du sang est aménagé, dont l'une des extrémités est reliée audit orifice d'entrée (4) dudit moyen de séparation de plasma (1) et dont l'autre extrémité est reliée audit orifice de sortie (2) dudit moyen (1) de séparation de plasma, le sang concentré par ledit moyen (1) de séparation de plasma s'écoulant dans ledit circuit destiné à la circulation du sang;
un moyen de circulation (3) est installé en vue de faire circuler le sang à l'intérieur dudit circuit destiné à la circulation du sang, dudit orifice de sortie (2) audit orifice d'entrée (4) dudit moyen (1) de séparation de plasma;
ledit moyen (8) formant réservoir de sang est relié audit circuit pour la circulation du sang du côté de la sortie de celui-ci pour accumuler ou recueillir une quantité excédentaire de sang concentré, qui dépasse la capacité d'écoulement dudit circuit destiné à la circulation du sang;
ledit moyen de transport de sang est raccordé entre ladite aiguille commune (8) unique et ledit circuit destiné à la circulation du sang;
et le moyen (1) de séparation de plasma comporte en outre un moyen (19) destiné à faire varier la surface de la section transversale des passages pour le sang, définis à l'intérieur.

2. Appareil selon la revendication 7, dans lequel ledit moyen (8) formant réservoir le sang comporte une poche étanche fabriquée en résine de synthèse souple.

3. Appareil selon la revendication 7, comprenant en outre au moins deux pompes (7, 3), l'une (7) destinée à entraîner ledit moyen de transport de sang et l'autre (3) destinée à entraîner ledit moyen de circulation.

4. Appareil selon la revendication 7, comprenant en outre aux moins deux récipients (10, 11) contenant du liquide, et un moyen d'alimentation en liquide associé fonctionellement audit moyen de transport de sang (7) pour fournir de façon sélective audit moyen de transport de sang un liquide provenant desdits récipients contenant du liquide selon un rapport prédéterminé.

5. Appareil selon la revendication 12, dans lequel le liquide contenu dans l'un (10) desdits récipients renfermant du liquide est un anticoagulant, et le liquide contenu dans l'autre (11) desdits récipients renfermant du liquide est un liquide de remplacement en quantité équivalente à la quantité de plasma qui a été séparé.

6. Appareil pour la séparation du plasma comprenant:
une aiguille commune (18) unique destinée à l'introduction du sang et au retour du sang, un moyen (1) de séparation de plasma, du type membrane, comportant un orifice d'entrée (4) pour y introduire le sang et un orifice de sortie (2) pour en évacuer le sang concentré,
un moyen de transport de sang qui comporte une pompe (7) de transport de sang, susceptible de tourner de façon réversible pour introduire et évacuer le sang par ladite unique aiguille commune,
et un moyen formant réservoir (8) relié audit moyen de séparation de plasma pour accumuler ou receuillir temporairement le sang concentré, caracterise en ce que
un circuit pour la circulation du sang est aménagé, dont l'une des extrémités est reliée audit orifice d'entrée (4) dudit moyen (1) de séparation de plasma et dont l'autre extrémité est reliée audit orifice de sortie (2) dudit moyen (1) de séparation de plasma, le sang concentré par ledit moyen (1) de séparation de plasma s'écoulant dans ledit circuit destiné à la circulation du sang;
un moyen de circulation (3) est installé en vue de faire circuler le sang à l'intérieur dudit circuit destiné à la circulation du sang, dudit orifice de sortie (2) audit orifice d'entrée (4) dudit moyen de séparation (1);

ledit moyen (8) formant réservoir de sang est installé dans ledit circuit destiné à la circulation du sang pour accumuler ou recueillir temporairement le sang prélevé par ladite unique aiguille commune (18) et concentré par ledit moyen de séparation de plasma;

et ledit moyen de transport de sang est raccordé entre ladite unique aiguille commune (18) et ledit moyen (8) formant réservoir de sang

et ledit moyen (1) de séparation de plasma comporte en outre un moyen destiné à faire varier la surface de la section transversale des passages pour le sang, définis à l'intérieur.

7. Appareil selon la revendication 6, dans lequel ledit moyen (8) formant réservoir de sang comporte une poche étanche fabriquée en résine de synthèse souple.

8. Appareil selon la revendication 6, comprenant en outre au moins deux pompes (7, 3), l'une destinée à entraîner ledit moyen de transport de sang et l'autre destinée à entraîner ledit moyen de circulation.

9. Appareil selon la revendication 6, comprenant en outre au moins deux récipients (10, 11) contenant du liquide et un moyen d'alimentation en liquide fonctionnellement associé audit moyen de transport de sang pour fournir de façon sélective audit moyen de transport de sang un liquide provenant desdits récipients contenant du liquide, selon un rapport prédéterminé.

10. Appareil selon la revendication 9, dans lequel le liquide contenu dans l'un (10) desdits récipients contenant du liquide est un anticoagulant, et le liquide contenu dans l'autre (11) desdits récipients contenant du liquide est un liquide de replacement en quantité équivalente à la quantité de plasma qui a été séparé.

# F I G. 1 (A)

# F I G. 1 (B)

BLOOD RETURN

BLOOD COLLECTION

SEPARATOR

# F I G. 1 (C)

*F I G. 1* (D)

# F I G. 2 (A)

# F I G. 2 (B)

FIG. 3

BLOOD COLLECTION | BLOOD RETURN | BLOOD COLLECTION | BLOOD RETURN | BLOOD COLLECTION | BLOOD RETURN

AMOUNT OF SEPARATED PLASMA

500

510

OPERATING TIME

EP 0 171 749 B1

FIG. 4

FIG. 5
PRIOR ART

STIRRER

SEPARATOR

## FIG. 6 (A)

## FIG. 6 (B)

## FIG. 8 (A)

## FIG. 8 (B)

EP 0 171 749 B1

F I G. 7

EP 0 171 749 B1

F I G. 9

F I G. 10

PRIOR ART

SEPARATION

STIRRER 22

EP 0 171 749 B1

## F I G. 11 (A)

## F I G. 12 (A)

## F I G. 11 (B)

## F I G. 12 (B)

EP 0 171 749 B1